# EUROPEAN PATENT SPECIFICATION

(11) **EP 0 436 009 B1**
(45) Date of publication and mention of the grant of the patent: **06.04.1994**
(21) Application number: 90911640.2
(22) Date of filing: 13.07.1990
(51) Int. Cl.: D04H 1/42, D04H 1/54

(54) **ABSORBENT FIBROUS WEB CONTAINING CELLULOSE-CONTAINING RECYCLED MATERIAL**
ABSORBIERENDES FASERBAND MIT WIEDERVERWENDETEM ZELLULOSEMATERIAL
MATIERE FIBREUSE ABSORBANTE A BASE DE MATERIAU CELLULOSIQUE RECYCLE

(30) Priority: 21.07.1989 CH 2744/89
(43) Date of publication of application: 10.07.1991
(73) Proprietor: Leone, Demetrio, CH-8437 Zurzach (CH)
(72) Inventor: Leone, Demetrio, CH-8437 Zurzach (CH)
(74) Representative: Breiter, Heinz
(86) International application number: EP9001151
(87) International publication number: WO9101396

(56) References cited:
- EP-A- 53 345
- EP-A- 227 914
- WO-A-86/02116

## Description

The present invention relates to a process for producing an absorbent fibrous web containing a cellulose-containing recycled material, in particular paper and/or textiles. The invention further relates to the use of the process for producing shaped absorbent pads and to the use of the web as an absorbent core.

It is known to produce liquid-absorbing pads or mats by mechanical defibration of a cellulose pulp in a dry process, and then converting the defibrated material into the desired pad or mat form.

In a particular process described in EP-B1-0 202 237, wetting-resistant or coated waste material is used as cellulose-containing starting material. If necessary, it is cut to a suitable size for defibration. During or immediately prior to defibration, a mixture of water and a surfactant is added to the paper waste in order to restore the absorptive capacity of the cellulose fibres. Here the amount of mixture is up to 25 % by volume, based on the volume of the wastepaper.

Although the absorptive capacity of the cellulose fibres is restored with the mixture of water and a surfactant, pads or mats produced by the process of the above-cited European Patent neither exhibit the customary level of wear comfort nor are of the necessary quality for a competitive product.

It is an object of the present invention to provide a process of the type mentioned at the beginning whereby it is possible, despite the use of inexpensive starting materials, to produce in an economical manner high-quality webs which are also suitable for manufacturing shaped absorbent cores of high wear comfort.

This object is achieved according to the present invention by a process which is characterized in that the recycled material is defibrated, and the cellulose-containing recycled fibres are cleaned and mixed with fibres formed from at least one thermoplastic to give a homogeneous pulp, which is formatted and subjected to a heat treatment to bond together the fibres at many points, producing a compact layer of cellulose-containing recycled fibres and plastics fibres which exhibits networklike interbonding and increased yet elastic shape retention.

The defibration of recycled material, in particular paper and/or textiles, is effected in a conventional manner and corresponds in principle to the defibration of round timber chips in a defibrator. If necessary, the recycled material must be trimmed before defibration at least to the maximum size suitable for the machine. It is possible to use any cellulose-containing material obtained after use and/or before processing.

The cellulose-containing recycled fibres obtained from the recycled material are cleaned in a conventional manner using at least one physical and/or chemical process. In the course of this process, any reduced absorptivity of the cellulose-containing recycled fibres is substantially restored.

The cellulose-containing recycled fibres and the plastics fibres are intimately mixed, preferably in a ratio of from 30:1 to 4:1, to form a homogeneous fibre blend.

Before mixing into the cellulose-containing recycled fibres, the plastics fibres are advantageously treated with a wetting agent. This surface-active agent reduces the surface tension of water, thereby facilitating the penetration of body fluid to the web and accelerating the spreading of the fluid on the surface of the plastic fibres. The wetting agents to be used are known to the person skilled in the art. A specific example of a wetting agent is Triton X-100 from DUPONT.

The plastics fibres used preferably consist of a polyolefin or of a polyester. Examples of materials of plastics fibres are polyethylene polypropylene, copolymers of ethylene and propylene and also copolymers of propylene and butylene.

The plastics fibres mixed with the cellulose-containing recycled fibres generally have a diameter of 1 - 40 µm, in particular 5 - 20 µm.

It has proved to be particularly advantageous to mix cellulose-containing fibres with fibrillated polyethylene. A product from DUPONT known as "PULPLUS" and another product, "PULPEX" from Hercules, consist for example of fine, 1 - 20 µm thick fibrils which form numerous bonds in the course of the heat treatment through thermobonding. In addition to the very small diameter, these plastics fibres have inter alia the following advantageous properties:
- high strength and orientation
- ready meltability
- ready processibility
- high specific surface area
- chemical inertness
- environmental safeness
- inexpensiveness.

Absorbent pads produced from the fibre blend by thermobonding have the following advantageous properties:
- greater retention capacity for liquids
- improved retention capacity for pulverulent superabsorbers
- increased shape retention with retained elasticity
- higher strength before and after wetting
- great reduction in specific gravity.

The heat treatment, for example in a hot air stream, to bond the fibres together by local fusion is preferably effected at a temperature just below the combustion temperature of cellulose, in particular at 200-220°C. For both the process and the apparatus reference is made to the applicant's Swiss Patent 675829.

The cellulose-containing recycled fibres are advantageously mixed not only with plastics fibres but also with a chemical absorbent, in particular with a superabsorber. Alternatively, the absorbent, in particular superabsorber, can also be homogeneously dispersed in the web after the heat treatment.

Superabsorbers are highly absorbent polymers which can bind a multiple of their weight in salt-containing water or urine and in so doing form a gel which remains stable even under the action of pressure. This retention is a particularly important property for nappies, linings, sanitary towels, tampons and the like. The rate of absorption of a superabsorber depends in the main on its particle size; that is, smaller particles have a larger total superabsorber surface area and therefore absorption or gel formation take place significantly faster. The absorbency of an absorbent layer is set using different superabsorbers and/or different amounts of superabsorbers.

The superabsorbers used are for example the following products:
- Aridall 1125: from Chemdal, U.S.A.
- Drytech 510: from Dow Chemical, U.S.A.
- PR 9910 S: from Floerger, U.S.A.
- FAVOR 922 SK: from Stockhausen, FRG

If an absorbent contains thermoplastics fibres, for example fibrillated polyethylene (cf. for example US-A1-4458042), the separate addition of plastics fibres can be dispensed with. The plastics fibres present in the absorbent are capable of effecting adequate consolidation of the web in the course of the heat treatment.

The use of the process according to the present invention is the manufacture of shaped absorbent cores by contacting the ready-produced mixed pulp with at least one hot surface. This hot surface can take the form for example of a hot bed, a hot roll or a moving, endless hot belt.

According to the present invention, the web produced by the process is used as an absorbent core in nappies and disposable nappies for small children and adult patients, incontinence linings, sanitary towels, panty-linings, bed linings and disposable absorbent compresses. However, the web can also be used in general for medical and industrial purposes, in particular for protection pads against the action of water and for absorbing existing water.

## Claims

1. A process for producing an absorbent fibrous web containing a cellulose-containing recycled material, in particular paper and/or textiles,
characterized in that
the recycled material is defibrated, and the cellulose-containing recycled fibres are cleaned and mixed with fibres formed from at least one thermoplastic to give a homogeneous pulp, which is formatted and subjected to a heat treatment to bond together the fibres at many points, producing a compact layer of cellulose-containing recycled fibres and plastics fibres which exhibits networklike interbonding and increased yet elastic shape retention.

2. A process according to Claim 1, characterized in that the cellulose-containing recycled fibres and plastics fibres are mixed in a ratio of 30:1 to 4:1.

3. A process according to Claim 1 or 2, characterized in that the plastics fibres are treated with a wetting agent before mixing.

4. A process according to any one of Claims 1 - 3, characterized in that the cellulose-containing recycled fibres are mixed with fibres formed from a polyolefin or from a polyester.

5. A process according to any one of Claims 1 - 4, characterized in that the cellulose-containing recycled fibres are mixed with plastics fibres having a diameter within the range 1 - 40 µm, preferably 5 - 20 µm.

6. A process according to any one of Claims 1 - 5, characterized in that the cellulose-containing recycled fibres are mixed with fibrillated polyethylene.

7. A process according to any one of Claims 1 - 6, characterized in that the heat treatment is carried out at just below the combustion temperature of cellulose, preferably at 200 - 220°C.

8. A process according to any one of Claims 1 - 5, characterized in that the cellulose-containing recycled fibres are mixed with plastics fibres and a chemical absorbent, preferably a superabsorber, or a chemical absorbent, preferably a superabsorber, is homogeneously dispersed in the web after the heat treatment.

9. A process according to any one of Claims 1 - 8, for manufacturing shaped absorbent cores by contacting the formatted pulp with at least one hot surface.

10. The use of the fibrous web produced according to any one of Claims 1 - 9 as an absorbent core in nappies and disposable nappies for small children and adult patients, incontinence linings, sanitary towels, panty-linings and bed linings and for industrial and medical purposes, such as disposable absorbent compresses.

## Patentansprüche

1. Verfahren zur Herstellung eines saugfähigen Faservlieses mit zellstoffhaltigem Recyclingmaterial, insbesondere aus Papier und/oder Textilien,
dadurch gekennzeichnet, dass
das Recyclingmaterial zerfasert, die zellstoffhaltigen Recycling-Fasern gereinigt und mit Fasern aus wenigstens einem thermoplastischen Kunststoff zu einer homogenen Pulpe gemischt werden, welche formatiert und einer Wärmebehandlung zum Verbinden der Fasern an vielen Punkten unterworfen wird, wodurch eine netzartig verbundene, kompakte Schicht aus zellstoffhaltigen Recycling-Fasern und Kunststoffasern mit erhöhter, jedoch elastischer Formfestigkeit entsteht.

2. Verfahren nach Anspruch 1, dadurch gekennzeichnet, dass die zellstoffhaltigen Recycling-Fasern und die Kunststoffasern in einem Verhältnis von 30 : 1 bis 4 : 1 gemischt werden.

3. Verfahren nach Anspruch 1 oder 2, dadurch gekennzeichnet, dass die Kunststoffasern vor dem Einmischen mit einem Benetzungsmittel behandelt werden.

4. Verfahren nach einem der Ansprüche 1 - 3, dadurch gekennzeichnet, dass die zellstoffhaltigen Recycling-Fasern mit Kunststoffasern aus einem Polyolefin oder aus einem Polyester gemischt werden.

5. Verfahren nach einem der Ansprüche 1 - 4, dadurch gekennzeichnet, dass die zellstoffhaltigen Recycling-Fasern mit Kunststoffasern eines Durchmessers im Bereich von 1 - 40 µm, vorzugsweise 5 - 20 µm, gemischt werden.

6. Verfahren nach einem der Ansprüche 1 - 5, dadurch gekennzeichnet, dass die zellstoffhaltigen Recycling-Fasern mit fibrilliertem Polyäthylen gemischt werden.

7. Verfahren nach einem der Ansprüche 1 - 6, dadurch gekennzeichnet, dass die Wärmebehandlung bei einer knapp unterhalb der Verbrennungstemperatur von Zellulose liegenden Temperatur, vorzugsweise bei 200 - 220°C, durchgeführt wird.

8. Verfahren nach einem der Ansprüche 1 - 5, dadurch gekennzeichnet, dass die zellstoffhaltigen Recycling-Fasern mit Kunststoffasern und einem chemischen Absorptionsmittel, vorzugsweise mit einem Superabsorber, gemischt werden, oder ein chemisches Absorbtionsmittel, vorzugsweise ein Superabsorber, nach der Wärmebehandlung möglichst homogen im Vlies verteilt wird.

9. Anwendung des Verfahrens nach einem der Ansprüche 1 - 8, zur Herstellung von geformten Saugkernen durch Kontaktieren der formatierten Pulpe mit wenigstens einer heissen Fläche.

10. Verwendung des nach einem der Ansprüche 1 - 8 hergestellten Faservlieses als Saugkern in Windeln und Windelhöschen für Kleinkinder und kranke Erwachsene, Inkontinenzeinlagen, Monats- und Wöchnerinnenbinden, Slipeinlagen, Betteinlagen sowie für industrielle und medizinische Zwecke, wie Wegwerf-Saugkompressen.

## Revendications

1. Procédé de production d'un tissu fibreux absorbant contenant une matière recyclée contenant de la cellulose, notamment papier et/ou textiles, caractérisé en ce que la matière recyclée est défibrée et que les fibres recyclées contenant de la cellulose sont nettoyées et mélangées avec des fibres faites en au moins une matière thermoplastique, pour donner une pâte homogène qui est préalablement transformée en mat de fibres et soumise à un traitement thermique destiné à agglomérer les fibres en de nombreux points, produisant une couche compacte de fibres recyclées contenant de la cellulose et de fibres en matière plastique et qui présente une inter-agglomération de type réseau et une conservation accrue mais élastique de sa forme.

2. Procédé selon la revendication 1, caractérisé en ce que les fibres recyclées contenant de la cellulose et les fibres en matière plastique sont mélangées dans un rapport de 30:1 à 4:1.

3. Procédé selon la revendication 1 ou 2, caractérisé en ce que les fibres en matière plastique sont traitées à l'aide d'un agent mouillant avant le mélange.

4. Procédé selon l'une quelconque des revendications 1 à 3, caractérisé en ce que les fibres recyclées contenant de la cellulose sont mélangées à des fibres en polyoléfine ou polyester.

5. Procédé selon l'une quelconque des revendications 1 à 4, caractérisé en ce que les fibres recyclées contenant de la cellulose sont mélangées avec des fibres en matière plastique de diamètre compris dans la plage de 1 à 40 µm, de préférence de 5 à 20 µm.

6. Procédé selon l'une quelconque des revendications 1 à 5, caractérisé en ce que les fibres recyclées contenant de la cellulose sont mélangées avec du polyéthylène fibrillé.

7. Procédé selon l'une quelconque des revendications 1 à 6, caractérisé en ce que le traitement thermique est mis en oeuvre à une température immédiatement inférieure à la température de combustion de la cellulose, de préférence entre 200 et 220°C.

8. Procédé selon l'une quelconque des revendications 1 à 5, caractérisé en ce que les fibres recyclées contenant de la cellulose sont mélangées avec des fibres en matière plastique et avec un absorbant chimique, de préférence un superabsorbant, ou en ce qu'un absorbant chimique, de préférence un superabsorbant, est dispersé de façon homogène dans le tissu après le traitement thermique.

9. Procédé selon l'une quelconque des revendications 1 à 8, destiné à la fabrication de noyaux absorbants façonnés, par mise en contact de la pâte transformée préalablement en mat avec au moins une surface chaude.

10. Emploi du tissu fibreux produit selon l'une quelconque des revendications 1 à 9, comme noyau absorbant de couches et couches à jeter destinées à l'enfant et à l'adulte, de doublures anti-incontinence, de garnitures hygiéniques, de doublures de culottes et de draps de lit ainsi qu'à des fins industrielles et médicales, par exemple comme compresses absorbantes à jeter.
